# EUROPEAN PATENT SPECIFICATION

(11) **EP 1 105 829 B1**
(45) Date of publication and mention of the grant of the patent: **13.11.2002**
(21) Application number: 99939518.9
(22) Date of filing: 11.08.1999
(51) Int. Cl.: G06K 7/10

(54) **IMAGE ACQUISITION APPARATUS**
BILDERFASSUNGSGERÄT
APPAREIL D'ACQUISITION D'IMAGES

(30) Priority: 19.08.1998 GB 9817982; 24.12.1998 GB 9828668
(43) Date of publication of application: 13.06.2001
(73) Proprietor: Oxoid Limited, Basingstoke, Hampshire RG24 8PW (GB)
(72) Inventor: HOLLEY, John, Ernest, Foster, N. Westerham, Kent TN16 2AU (GB)
(74) Representative: Roberts, David Leslie
(86) International application number: GB9902645
(87) International publication number: WO00011593

(56) References cited:
- EP-A- 0 326 796
- EP-A- 0 745 950
- US-A- 4 636 846
- US-A- 5 618 729
- PATENT ABSTRACTS OF JAPAN vol. 012, no. 125 (P-691), 19 April 1988 (1988-04-19) & JP 62 250343 A (JAPAN TOBACCO INC), 31 October 1987 (1987-10-31)

## Description

### Field of the Invention

This invention relates to apparatus for acquiring an image of the contents of a container for subsequent analysis. The apparatus is especially, but not exclusively, applicable to systems for use in antibiotic susceptibility testing of micro-organisms.

### Background to the Invention

US-A-5 618 729 discloses an image acquisition apparatus for acquiring an image of the contents of a petri plate. The apparatus comprises a light source, a video camera and an image processor. The petri plate is held within the field of view of the camera by a holding fixture.

Antibiotic susceptibility testing has for many years been used as a means for identifying particular groups or species of micro-organisms, or for identifying an antibiotic type or dose level most appropriate for dealing with a particular clinical infection.

Usually, six or eight discs, carrying different types of antibiotic or the same type of antibiotic but in different concentrations, are placed in a circular array on a petri dish. The dish contains a layer of growth medium, such as agar gel, to which a material containing a micro-organism to be analysed is applied.

The antibiotic diffuses out of each disc into the surrounding growth medium and establishes a radial concentration gradient around the disc. The diameter of the zone of inhibited micro-organism growth around the disc is indicative of the relative susceptibility of the micro-organisms to the antibiotic on that disc. The detailed morphology of the zone can also provide information on the species or genus of micro-organism presence.

There have been various proposals for automating the analysis of the contents of the petri dishes, especially the inhibition zones, involving the use of an overhead video camera connected to a data processing device.

The petri dishes used in such tests are commonly provided with identification markings. For example, in hospitals, the patient from whom the micro-organism in a petri dish has been extracted is identified by means of a bar code applied to the side of the petri dish.

Since the sides of the petri dishes are vertical, it is difficult if not impossible for the bar codes to be read by an overhead camera of an automatic analysis system.

### Summary of the Invention

According to a first aspect of the invention, there is provided image acquisition apparatus for acquiring an image of the contents of a container, a side of the container carrying identification markings, the apparatus comprising overhead image capture means for obtaining an image of the contents of the container situated under the image capture means, receiving means for receiving the container and positioning it within the field of view of the image capture means, the apparatus further comprising light deflecting means for deflecting light from said identification markings to the image capture means to enable the latter also to obtain an image of said markings.

Thus, the deflection means enables the apparatus co acquire an image not only of the contents of the container, but also of the identification markings. If the apparatus is used in an AST system, a signal representative of that image can be supplied to a data processor which analyses the contents of the container (for example a petri dish) to determine the size and shape of the zones of inhibition around the carrier discs, and also to read the identification markings.

Preferably, the light deflecting means comprises a reflecting means positioned, on the receiving means, adjacent a location at which a container is to be received.

The reflecting means is preferably so inclined, relative to the receiving means, as to reflect light which is incident thereon from a horizontal direction upwards into the image capture means.

Thus, if the apparatus is to be used with petri dishes having identification markings on their vertical sides, the reflecting means may be inclined at an angle of 45°, but is preferably inclined at an angle of 30° to the horizontal or (alternatively) at 30° to the vertical.

Preferably, the reflecting means is annular.

This enables the reflecting means to surround a container, such as a petri dish, so that the image of identification markings on the container wall can be reflected into the image capture means regardless of the orientation of the container.

Preferably, the receiving means comprises a support having a well, recess or depression for receiving and accommodating said container, wherein said reflecting means is incorporated into the wall of the well.

Preferably, the well wall is frusto-conical, thereby to provide said inclination of the reflecting means, and the support may to advantage be formed of metal, at least part of the wall of the well having been polished to provide said reflecting means.

Preferably, the well is one of a plurality of such wells in the support, each for accommodating a respective container.

The image capture means conveniently comprises a scanner.

The invention also lies in apparatus as herein above described, and a plurality of containers, such as petri dishes, each carrying identification markings.

Preferably, the output of the image capture means is connected to data processing means for identifying a container on the receiving means and analysing the image of the container contents.

Preferably, the apparatus is adapted to perform antibiotic susceptibility tests to identify a particular micro-organism grown on a culture in a container.

### Brief Description of the Drawings

The invention will now be described, by way of example only, with reference to the accompanying drawings in which:
Figure 1 is a schematic view of apparatus in accordance with the invention;
Figure 2 is a more detailed sectional side elevation of part of the apparatus shown in Figure 1;
Figure 3 is a perspective view of a petri dish and part of a support for the apparatus;
Figure 4 is a perspective view of the support; and
Figure 5 is a plan view of part of the support with a petri dish therein.

### Detailed Description

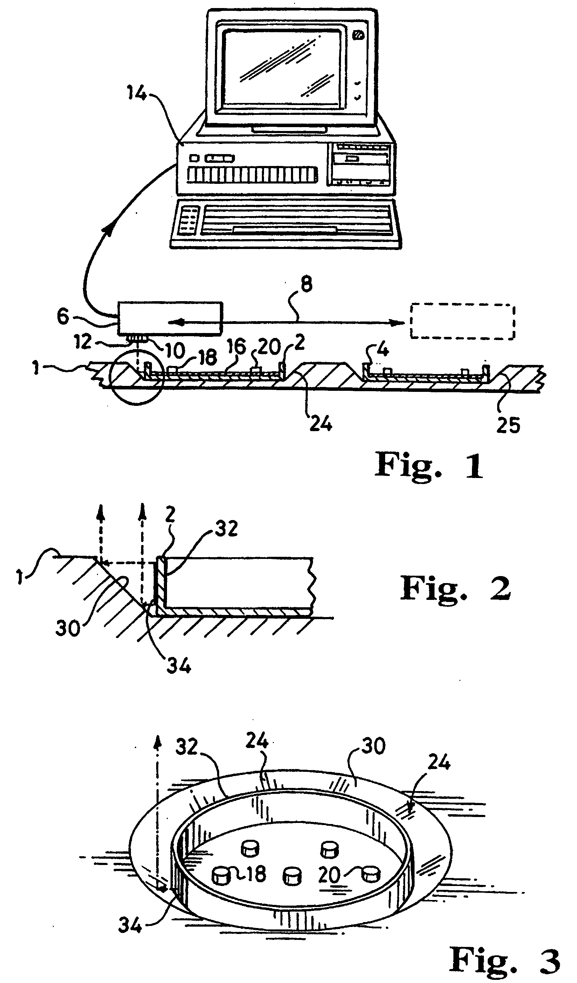

With reference to Figure 1, an embodiment of apparatus in accordance with the invention comprises a metal carrier plate 1 which acts as support means for a number of petri dishes, for example 2 and 4. The plate 1 is situated underneath an image scanner which is of the same type as a conventional flat bed scanner, and includes a scanning head 6. For the sake of clarity, only the scanning head 6 of the scanner is shown in Figure 1. The head 6 is mounted in the scanner via a suitable drive for causing the head to sweep over the whole plate 1 in the directions indicated by the arrow 8. The head 6 includes an output 10 which directs a beam of light vertically downwards onto the support 1, and hence the petri dishes. Light reflected from the support 1 and petri dishes is received at an input 12 for focusing onto a light sensitive receptor in the head 6 for converting the received light into an electrical signal representative of the image of the plate and dishes thereon captured by the scanning head 6.

That signal is fed to a computer 14, which is arranged to convert the signal into a digital data stream, and to manipulate and analyse the image represented thereby. The apparatus is intended for use in AST analysis of micro-organisms grown on an agar gel layer such as a layer 16 in the dish 2. The growth is affected by antibiotics initially carried by carrier discs, two of which are shown at 18 and 20 on the layer 16. There are various ways in which the analysis of the image of the areas of growth (or inhibition of growth) can be performed by the computer 14 (but these are not the subject of the present invention).

The computer may, for example, measure the area of the zone of inhibited growth of micro-organisms around the disc and, in some cases, may be operable to send character codes on the discs (possibly with the aid or orientation markings also on the discs) to identify the type and/or concentration of reagent (eg an antibiotic) carried by the disc.

However, a description of how a computer of an AST testing apparatus can analyse acquired images is provided in the present Applicants' PCT Patent Application published under No. WO99/02645.

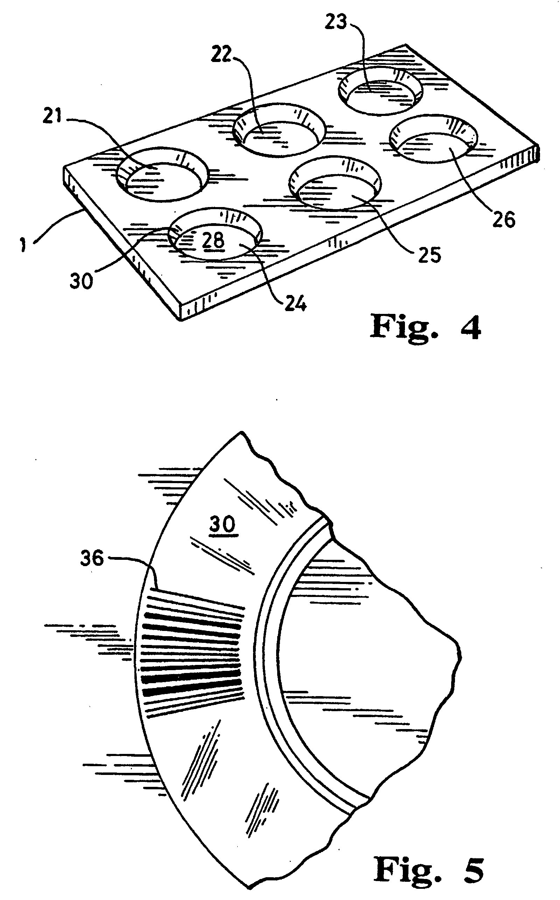

With reference to Figure 4, the upper surface of the plate 1 is provided with six circular wells recesses or depressions 21-26, each for accommodating a respective petri dish. The wells are arranged in two rows of three. Since the wells are identical, only the well 24 will be described. This well has a circular base 28 from which a frusto-conical wall 30 rises. The wall 30 flares from the bottom to the top of the well 24, and is polished so as to define an annular reflecting surface which in this embodiment makes an angle of approximately 45° with the base 28.

In another embodiment of the invention, the reflecting means is inclined at an angle of 30° to the vertical. In a further embodiment, the reflecting means is inclined at an angle of 30° to the horizontal.

As can be seen from Figures 1-3, the well 24 accommodates the petri dish 2 which, like the other petri dishes, has a vertical, cylindrical side wall 32, the exterior of which carries identification markings in the form of a bar code 34 (Figures 2 and 3) printed on a self adhesive label stuck to the wall 32.

The scanner head is not able to obtain images of the vertical side wall of the petri dish 2, and therefore cannot directly read the bar code 34 from the latter. However, the image of the bar code 34 is reflected from the wall 30 as shown by the broken lines in Figures 2 and 3. Accordingly, the scanning head 6 can form an image of the reflection of the bar code 34 in the wall 30 as shown in Figure 5, in which the reflected image of the bar code 34 is denoted by the reference numeral 36.

The image 36 is laterally inverted by virtue of its reflection in the surface 30. However, this can be handled by the image processing software in the computer 14 which can reverse the direction of reading of the bar code 34. Alternatively, the bar code itself can be laterally inverted compared with the norm so that it can be read by the computer 14 as if it were a conventionally orientated bar code.

## Claims

1. Image acquisition apparatus for acquiring an image of the contents of a container (2, 4), a side (32) of the container carrying identification markings (34), the apparatus comprising overhead image capture means (6, 14) for obtaining an image of the contents of a container situated under the image capture means, receiving means (1, 21-26, 28) for receiving the container and positioning it within the field of view of the image capture means, the apparatus further comprising light deflecting means (30) for deflecting light from said identification markings (34) to the image capture means (6, 14) to enable the latter also to obtain an image of said markings.

2. Apparatus according to claim 1, in which the light deflecting means (30) comprises a reflecting means positioned, on the receiving means, adjacent a location at which a container is to be received.

3. Apparatus according to claim 2, in which the reflecting means (30) is so inclined, relative to the receiving means, as to reflect light which is incident thereon from a horizontal direction upwards into the image capture means.

4. Apparatus according to claim 3, in which the reflecting means (30) is inclined at an angle of 45° to enable the scanning means to read identification markings on the vertical sides of containers for use therewith.

5. Apparatus according to claim 3, in which the reflecting means is inclined at an angle of 30° to the horizontal or 30° to the vertical, to enable the apparatus to read the markings on the vertical sides of containers for use therewith.

6. Apparatus according to any of claims 2 to 5, in which the reflecting means is annular, the arrangement being such that, in use, the reflecting means surrounds a container in the receiving means of the apparatus.

7. Apparatus according to any of claims 2 to 6, in which the receiving means comprises a support (1) having a well (21-26), recess or depression for receiving and accommodating said container, wherein said reflecting means is incorporated into the wall of the well.

8. Apparatus according to claim 7 when appended to claim 3, in which the well wall is frusto-conical, thereby to provide said inclination of the reflecting means.

9. Apparatus according to claim 7 or claim 8, in which the support (1) is formed of metal, at least part of the wall of the well having a smooth surface to provide said reflecting means.

10. Apparatus according to any of claims 7 to 9, in which the well is one of a plurality of such wells in the support, each for accommodating a respective container.

11. Apparatus according to any of the preceding claims, in which image capture means comprises a scanner.

12. Analysis apparatus comprising image acquisition apparatus according to any of the preceding claims and a plurality of containers (2, 4), such as petri dishes, each carrying respective identification markings.

13. Analysis apparatus according to claim 12, in which the output of the image capture means (6) is connected to data processing means (14) for identifying a container on the receiving means and analysing the image of the container contents.

14. Analysis apparatus according to claim 12 or claim 13, in which the apparatus is so arranged to be operable to perform antibiotic susceptibility tests to identify a particular micro-organism grown on a culture in a container.

## Patentansprüche

1. Bildgewinnungseinrichtung zum Gewinnen eines Bildes des Inhaltes eines Behälters (2, 4), dessen eine Seite (32) Identifikationsmarkierungen (34) trägt, wobei die Einrichtung ein Overhead-Bilderfassungsmittel (6, 14) zum Erzielen eines Bildes des Inhaltes eines unter dem Bilderfassungsmittel angeordneten Behälters und Aufnahmemittel (1, 21 - 26, 28) zum Aufnehmen des Behälters und zu seiner Positionierung im Blickfeld des Bilderfassungsmittels aufweist, und die Einrichtung weiter Lichtablenkmittel (30) zum Ablenken des Lichtes von den Identifikationsmarkierungen (34) zum Bilderfassungsmittel (6, 14) aufweist, damit das letztere auch ein Bild der Markierungen erhalten kann.

2. Einrichtung nach Anspruch 1, bei der das Lichtablenkmittel (30) ein auf dem Aufnahmemittel an einer Stelle, an der ein Behälter aufzunehmen ist, positioniertes Reflexionsmittel aufweist.

3. Einrichtung nach Anspruch 2, bei der das Reflexionsmittel (30) gegenüber dem Aufnahmemittel so geneigt ist, daß Licht, das aus einer horizontalen Richtung nach oben in das Lichterfassungsmittel eindringt, reflektiert wird.

4. Einrichtung nach Anspruch 3, bei der das Reflexionsmittel (30) unter einem Winkel von 45° geneigt ist, so daß das Abtastmittel Identifikationsmarkierungen auf den vertikalen Seiten der Behälter zur Verwendung mit diesen lesen kann.

5. Einrichtung nach Anspruch 3, bei der das Reflexionsmittel unter einem Winkel von 30° gegenüber der Horizontalen oder 30° gegenüber der Vertikalen geneigt ist, so daß die Einrichtung die Markierungen auf den vertikalen Seiten der Behälter zur Verwendung mit diesen lesen kann.

6. Einrichtung nach irgendeinem der Ansprüche 2 bis 5, bei der das Reflexionsmittel ringförmig und die Anordnung so getroffen ist, daß das Reflexionsmittel bei Verwendung einen Behälter in dem Aufnahmemittel der Einrichtung umschließt.

7. Einrichtung nach irgendeinem der Ansprüche 2 bis 6, bei der das Aufnahmemittel eine Auflage (1) mit einer Bohrung (21 - 26), einer Aussparung oder Vertiefung zum Aufnehmen und Unterbringen des Behälters aufweist, wobei das Reflexionsmittel in die Wand der Bohrung eingebaut ist.

8. Einrichtung nach Anspruch 7 bei Anschluß an Anspruch 3, bei der die Bohrungswand kegelstumpfförmig ist, um dadurch die Neigung des Reflexionsmittels auszubilden.

9. Einrichtung nach Anspruch 7 oder Anspruch 8, bei der die Aufnahme (1) aus Metall gebildet ist und mindestens ein Teil der Wand der Bohrung zum Ausbilden des Reflexionsmittels eine glatte Oberfläche aufweist.

10. Einrichtung nach irgendeinem der Ansprüche 7 bis 9, bei der die Bohrung eine aus einer Vielzahl solcher Bohrungen in der Auflage ist und jede Bohrung zum Unterbringen eines entsprechenden Behälters dient.

11. Einrichtung nach irgendeinem der vorhergehenden Ansprüche, bei der das Bilderfassungsmittel ein Abtaster ist.

12. Analyseeinrichtung mit einer Bildgewinnungseinrichtung nach irgendeinem der vorhergehenden Ansprüche und einer Vielzahl von Behältern (2, 4), wie zum Beispiel Petrischalen, von denen jede entsprechende Identifikationsmarkierungen trägt.

13. Analyseeinrichtung nach Anspruch 12, bei der der Ausgang des Bilderfassungsmittels (6) an ein Datenprozessormittel (14) zum Identifizieren eines Behälters auf dem Aufnahmemittel und zum Analysieren des Bildes des Behälterinhaltes angeschlossen ist.

14. Analyseeinrichtung nach Anspruch 12 oder Anspruch 13, bei der die Einrichtung so ausgebildet ist, daß sie zum Identifizieren eines bestimmten, auf einer Kultur in einem Behälter gewachsenen Mikroorganismus antibiotische Auffälligkeitsprüfungen durchführt.

## Revendications

1. Appareil d'acquisition d'images destiné à acquérir une image du contenu d'un conteneur (2, 4), un côté (32) du conteneur portant des marquages d'identification (34), l'appareil comprenant un moyen de prise d'images suspendu (6, 14) afin d'obtenir une image du contenu d'un conteneur situé sous le moyen de prise d'images, un moyen de réception (1, 21 à 26, 28) destiné à recevoir le conteneur et à le positionner dans le champ de vision du moyen de prise d'images, l'appareil comprenant de plus un moyen de déviation de la lumière (30) destiné à dévier la lumière provenant desdits marquages d'identification (34) vers le moyen de prise d'images (6, 14) afin de permettre à ce dernier d'obtenir aussi une image desdits marquages.

2. Appareil selon la revendication 1, dans lequel le moyen de déviation de la lumière (30) comprend un moyen réfléchissant positionné sur le moyen de réception, adjacent à un emplacement auquel on doit recevoir le conteneur.

3. Appareil selon la revendication 2, dans lequel le moyen réfléchissant (30) est incliné, par rapport au moyen de réception, de façon à réfléchir vers le haut la lumière qui est incidente sur lui à partir d'une direction horizontale dans le moyen de prise d'image.

4. Appareil selon la revendication 3, dans lequel le moyen réfléchissant (30) est incliné selon un angle de 45° pour permettre au moyen de numérisation de lire les marquages d'identification sur les côtés verticaux des conteneurs pour leur utilisation.

5. Appareil selon la revendication 3, dans lequel le moyen réfléchissant est incliné selon un angle de 30° par rapport à l'horizontale ou de 30° par rapport à la verticale, afin de permettre à l'appareil de lire les marquages d'identification sur les côtés verticaux des conteneurs pour leur utilisation.

6. Appareil selon l'une quelconque des revendications 2 à 5, dans lequel le moyen réfléchissant est annulaire, la disposition étant telle qu'en utilisation, le moyen réfléchissant entoure un conteneur dans le moyen de réception de l'appareil.

7. Appareil selon l'une quelconque des revendications 2 à 6, dans lequel le moyen de réception comprend un support (10) présentant un puits (21 à 26), un évidement ou un creux destiné à recevoir et à loger ledit conteneur, dans lequel ledit moyen réfléchissant est incorporé dans la paroi du puits.

8. Appareil selon la revendication 7 lorsqu'elle est annexée à la revendication 3, dans lequel la paroi du puits est tronconique, ce qui a pour effet de procurer ladite inclinaison du moyen réfléchissant.

9. Appareil selon la revendication 7 ou la revendication 8, dans lequel le support (1) est constitué de métal, une partie au moins de la paroi du puits présentant une surface lisse pour procurer ledit moyen réfléchissant.

10. Appareil selon l'une quelconque des revendications 7 à 9, dans lequel le puits en est un parmi une pluralité de tels puits dans le support, chacun étant destiné à loger un conteneur respectif.

11. Appareil selon l'une quelconque des revendications précédentes, dans lequel le moyen de prise d'images comprend un numériseur.

12. Appareil d'analyse comprenant l'appareil d'acquisition d'images selon l'une quelconque des revendications précédentes et une pluralité de conteneurs (2, 4), tels que des boîtes de Pétri, portant chacun des marquages d'identification respectifs.

13. Appareil d'analyse selon la revendication 12, dans lequel la sortie du moyen de prise d'images (6) est connectée au moyen de traitement des données (14) afin d'identifier un conteneur sur le moyen de réception et d'analyser l'image du contenu du conteneur.

14. Appareil d'analyse selon la revendication 12 ou la revendication 13, dans lequel l'appareil est disposé afin de pouvoir fonctionner pour effectuer des tests de sensibilité aux antibiotiques pour identifier un micro-organisme particulier croissant sur une culture dans un conteneur.
